# DEMANDE DE BREVET EUROPEEN

(11) **EP 1 870 136 A1**
(43) Date de publication de la demande: **26.12.2007**
(21) Numéro de dépôt: 06290953.6
(22) Date de dépôt: 12.06.2006
(51) Int. Cl.: A61Q 19/08, A61Q 19/00, A61K 8/97, A61K 8/35, A61K 8/34

(54) **Utilisation cosmétique d'une cassumunarine, d'un arylbutenoide et/ou d'un extrait botanique en contenant**

(71) Demandeur: Chanel Parfums Beauté, 92521 Neuilly sur Seine Cedex (FR)
(72) Inventeur: Holderith, Serge, 06410 Biot (FR); Lasserre, Christelle M. S., Jersey City, NJ 07302 (US); Maestro, Yannick G., 13500 Martigues (FR)
(74) Mandataire: Renard, Emmanuelle

(57) **Abrégé**

La présente invention concerne un procédé cosmétique de soin de la peau, destiné à prévenir et/ou traiter au moins un signe cutané du vieillissement, comprenant l'application topique sur la peau d'une composition renfermant au moins un actif choisi parmi une cassumunarine, un arylbuténoïde et un extrait botanique en contenant, tel qu'un extrait de *Zingiber cassumunar Roxb.*

## Description

La présente invention concerne un procédé cosmétique de soin de la peau, destiné à lutter contre au moins un signe cutané du vieillissement, comprenant l'application topique sur la peau d'une composition renfermant au moins une cassumunarine et/ou un arylbuténoïde et/ou un extrait botanique en contenant.

La peau est essentiellement constituée de trois couches, à savoir, en partant de la plus superficielle, l'épiderme, le derme et l'hypoderme.

L'épiderme est principalement constitué de kératinocytes, mais également de mélanocytes (intervenant dans la pigmentation de la peau) et de cellules de Langerhans, liés par des lipides intercellulaires. Sa fonction est de protéger le corps de l'environnement extérieur et d'assurer son intégrité, et en particulier de freiner la pénétration de micro-organismes ou de substances chimiques, et d'empêcher l'évaporation de l'eau contenue dans la peau qui conduirait à une déshydratation.

Le derme fournit un support solide à l'épiderme et assure également sa nutrition. Il est essentiellement constitué de fibroblastes et d'une matrice extracellulaire principalement constituée de collagène, d'élastine et de protéoglycanes.

Les fibres d'élastine sont en grande partie responsables de l'élasticité et de la fermeté de la peau. Elles sont d'abord synthétisées par les fibroblastes sous forme de tropoélastine portant des résidus lysine qui forment ensuite des microfibrilles d'élastine par liaison intramoléculaire.

Les fibres d'élastine ont tendance à se dégrader avec l'âge, en raison notamment d'une plus forte activité des élastases, résultant en un relâchement cutané. On observe également une dégradation des fibres élastiques (élastose) sous l'effet des UV.

Or, on recherche continuellement de nouveaux actifs cosmétiques permettant de prévenir ou lutter contre les signes cutanés du vieillissement et en particulier contre la perte de fermeté de la peau.

A cet égard, il est connu que certains actifs tels que l'acide ascorbique et ses dérivés (FR-2 847 816), les ginsenosides et extraits de *Panax* en contenant (FR-2 767 057) ou les extraits de *Polygonatum* (EP-1003538), par exemple, permettent d'augmenter la synthèse d'élastine par les fibroblastes et ainsi de lutter contre la perte de fermeté cutanée.

Toutefois, il subsiste toujours le besoin de proposer de nouveaux actifs cosmétiques permettant de lutter plus efficacement contre les signes cutanés du vieillissement et en particulier contre la perte de fermeté de la peau.

En outre, compte tenu de la recherche toujours croissante par les consommateurs de produits naturels renfermant le moins d'ingrédients synthétiques possibles, et des contraintes réglementaires de plus en plus lourdes pesant sur les composés issus de l'industrie chimique, il serait souhaitable que ces actifs cosmétiques soient d'origine végétale.

Or, la Demanderesse a démontré que certains actifs d'origine végétale, et notamment les phénylbuténoïdes et les cassumunarines, présentaient la propriété d'augmenter la synthèse d'élastine par les fibroblastes dermiques.

Les phénylbuténoïdes, extraits notamment des rhizomes de Bengle *(Zingiber cassumunar),* sont déjà connus comme inhibiteurs de cyclooxygenase-2 [Han Ah-reum et al., Chemical & Pharmaceutical Bulletin (2005) 53(11), 1466-1468], anti-inflammatoires et analgésiques [Ozaki Yukihiro et al., Chemical & Pharmaceutical Bulletin (1991), 39(9), 2353-6], anti-oxydants [Masu et al., Phytochemistry, Vol. 39, No. 2, pp. 459-461, 1995] et anti-fongiques [Ficker et al., Journal of Ethnopharmacology 85 (2003) 289-293].

L'effet anti-oxydant et anti-inflammatoire des cassumunarines extraites de *Zingiber cassumunar* a également été étudié [Masuda et al., JAOCS, Vol. 72, No. 9 (1995)].

Toutefois, il n'a encore jamais été suggéré, à la connaissance de la Demanderesse, d'utiliser des arylbuténoïdes, des cassumunarines ou des extraits botaniques en contenant, notamment certains extraits de Bengle, pour améliorer la fermeté de la peau par application topique.

Des extraits de Bengle sont, certes, classiquement utilisés dans la médecine traditionnelle indonésienne (Jamu) pour raffermir la peau. Toutefois, rien ne permet de penser qu'il s'agisse d'extraits contenant des arylbuténoïdes et/ou des cassumunarines, ni qu'ils aient été appliqués sur la peau.

Il a par ailleurs été suggéré d'utiliser des extraits aqueux ou alcooliques de cette plante (de préférence des rhizomes) pour lutter contre les signes cutanés du vieillissement en inhibant l'activité des collagénases et des élastases et en augmentant la production de collagène (JP2003-176230) ou en tant qu'agents blanchissants ou anti-pigmentants (JP09-169627 et JP09-071522). Ces extraits ne sont toutefois pas obtenus à l'aide de solvants organiques apolaires. Au contraire, l'extrait décrit dans la demande JP2003-176230 peut être obtenu après lavage à l'hexane des rhizomes, l'extrait hexanique étant par conséquent éliminé.

De même, il a été suggéré dans la demande JP2001-192339 d'utiliser des extraits de Bengle en tant qu'antagonistes de PAF, pour lutter contre la desquamation et la rugosité liées au syndrome de peau sèche, ou contre les irritations cutanées subséquentes, par exemple, au rasage. Toute partie de la plante et tout solvant d'extraction peuvent être utilisés.

L'art antérieur ci-dessus ne suggère donc pas précisément que des extraits de Bengle susceptibles d'être obtenus par extraction de rhizomes à l'aide d'au moins un solvant organique apolaire, tels que l'hexane, et renfermant par conséquent des arylbuténoïdes et/ou des cassumunarines, puissent présenter des propriétés d'activation de la synthèse d'élastine les rendant particulièrement bien adaptés à une application topique sur la peau en vue de prévenir et/ou traiter le relâchement cutané.

La présente invention a ainsi pour objet un procédé cosmétique de soin de la peau, destiné à prévenir et/ou traiter au moins un signe cutané du vieillissement, comprenant l'application topique sur la peau d'une composition renfermant au moins un actif choisi parmi une cassumunarine, un arylbuténoïde et un extrait botanique en contenant.

La présente invention a également pour objet l'utilisation cosmétique d'une cassumunarine et/ou d'un arylbuténoïde et/ou d'un extrait botanique en contenant, pour prévenir et/ou traiter au moins un signe cutané du vieillissement.

Par "extraits botaniques", on entend, au sens de la présente invention, des produits obtenus par extraction à l'aide de tout type de solvant (polaire ou apolaire, liquide ou gazeux) de toute partie d'une plante ou de la plante entière, en particulier à partir de ses feuilles, fleurs, rhizomes ou tiges.

Les cassumunarines constituent une classe de curcuminoïdes caractérisés par une structure renfermant un squelette 1-[2-(3-methoxy-4-hydroxy)phényl 6-(3,4-diméthoxyphényl]cyclohex-2-ényl penta-2,4-dién-1-one qui les différencie notamment de la curcumine. On distingue notamment les cassumunarines A, B et C.

Des exemples d'extraits botaniques contenant des cassumunarines sont les extraits de Bengle *(Zingiber cassumunar*)*.*

Par "arylbuténoïdes", on entend, au sens de la présente invention, les composés (non polaires) répondant à la formule (I) ci-dessous : 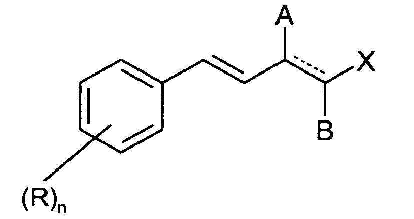 dans laquelle :
n désigne un nombre entier allant de 1 à 3 ;
R désigne un groupe hydroxy ou méthoxy, les groupes R pouvant être identiques ou différents dans le cas où n vaut 2 ou 3 ;
A désigne un atome d'hydrogène ou un groupe hydroxy et X désigne un atome d'hydrogène ou un groupe hydroxy ou acétoxy, ou A et X désignent chacun un atome de carbone et forment ensemble un cycle à six chaînons éventuellement insaturé, comportant éventuellement un ou plusieurs substituants (R'O)ₘAr où Ar est un groupe phényle, R' est un atome d'hydrogène ou un groupe acétyl et m est un nombre entier allant de 1 à 3 ; et
les pointillés désignent une liaison qui peut ou non être présente.

Dans la formule (I) ci-dessus, n est de préférence égal à 2. En outre, les groupes R sont de préférence disposés en positions méta et para sur le cycle. On préfère en outre que A et X désignent chacun un atome d'hydrogène. Enfin, dans le cas où A et X forment ensemble un cycle, on préfère qu'il soit substitué par un groupe diméthoxyphényle.

Les arylbuténoïdes selon la présente invention incluent également les isomères des composés de formule (I) .

Comme exemples d'arylbuténoïdes, on peut citer notamment ceux choisis parmi : la 4-(4-hydroxy-3-methoxyphenyl)-3-buten-2-one et les extraits de *Menta (Litsea sebifera)* en contenant, ainsi que le (E)-1-(3,4-diméthoxyphényl)but-1-ène, le (E)-4-(3,4-diméthoxyphényl)buta-1,3-diène, le (E)-4-(3,4-diméthoxyphényl)but-3-én-1-yl acétate, le (E)-4-(3,4-diméthoxyphényl)but-3-èn-1-ol, le (E)-3-hydroxy-1-(3,4-diméthoxyphényl)but-1-ène, le (E)-4-(4-hydroxy-3-méthoxyphényl)but-3-èn-1-yl acétate, le 4-(2,4,5-trimethoxyphenyl)but-1,3-diène et les extraits de *Zingiber cassumunar* en contenant.

On préfère utiliser dans la présente invention un extrait de Bengle ou *Zingiber cassumunar Roxb.* ou *Zingiber purpureum Roscoe,* qui renferme à la fois des cassumunarines et des arylbuténoïdes.

Les extraits botaniques éventuellement utilisés selon l'invention peuvent notamment être préparés par extraction de la plante considérée à l'aide d'au moins un solvant apolaire. Par cette expression, on entend un solvant ayant un indice de polarité inférieur à un, qui peut par exemple être choisi parmi : l'hexane, le cyclohexane, l'heptane et l'isooctane. Le solvant apolaire peut éventuellement être utilisé en mélange avec au moins un solvant polaire, ayant généralement un indice de polarité supérieur à 3.5, de préférence un solvant alcoolique tel que l'éthanol ou l'isopropanol, le ratio du solvant apolaire au solvant polaire étant de préférence compris entre 50:50 et 95:5 et allant de préférence de 70:30 à 90:10, plus préférentiellement de 75:25 à 85:15.

L'extraction peut être réalisée sur toute partie de la plante et, dans le cas du Bengle, plus particulièrement sur des rhizomes éventuellement séchés, qui peut être broyée ou réduite en morceaux de manière usuelle.

L'extraction est généralement réalisée en immergeant ou en agitant doucement le broyat dans un ou plusieurs des solvants cités ci-dessus à des températures allant, par exemple, de la température ambiante à 100°C, pendant une durée d'environ 30 min à 12 h.

La solution est alors de préférence filtrée afin d'éliminer les substances insolubles de la plante. On élimine également, le cas échéant, le solvant s'il s'agit d'un solvant volatil tel que, par exemple, l'éthanol, le méthanol, l'hexane ou le cyclohexane.

Cette étape d'extraction est usuelle dans le domaine des extraits de plantes, et l'homme du métier est à même d'en ajuster les paramètres réactionnels, sur la base de ses connaissances générales.

L'actif (molécule ou extrait botanique) utilisé dans la présente invention peut représenter de 0.00001 à 10 % en poids, de préférence de 0.0001 à 1 % en poids, et plus préférentiellement de 0.001 à 0.1 % en poids, par rapport au poids total de la composition.

Les signes cutanés du vieillissement visés dans la présente invention peuvent être des signes de vieillissement chronologique (intrinsèque) ou actinique (photo-vieillissement). L'invention vise plus particulièrement à lutter contre les signes cutanés liés au ralentissement de la synthèse, ou à la dégradation, de l'élastine et en particulier à prévenir et/ou traiter la perte de fermeté et/ou d'élasticité de la peau et/ou les rides.

De façon préférée, l'actif utilisé selon l'invention, ou la composition mise en oeuvre dans le procédé selon l'invention, sont appliqués sur une peau humaine, en particulier sur le corps et/ou le visage, notamment sur le contour des yeux. Ils sont toutefois préférentiellement appliqués sur le corps à l'exclusion du visage, et notamment sur les genoux, les cuisses, les hanches, les fesses, le ventre et/ou les bras. Ils sont avantageusement appliqués sur des sujets présentant des signes de relâchement cutané.

La composition renfermant cet actif peut être appliquée le matin et/ou le soir, de préférence le soir, sur les zones du corps à traiter, avantageusement après friction de ces zones pour stimuler la microcirculation. Cette composition peut ainsi, par exemple, être appliquée une fois par jour pendant quatre semaines.

La composition mise en oeuvre selon l'invention comprend généralement, outre l'actif décrit précédemment, un milieu physiologiquement acceptable et de préférence cosmétiquement acceptable, c'est-à-dire qui ne provoque pas de sensations d'inconfort (rougeurs, tiraillements, picotements...) inacceptables pour l'utilisateur.

Ce milieu contient généralement de l'eau et éventuellement d'autres solvants tels que l'éthanol.

La composition utilisée selon l'invention peut se présenter sous toute forme adaptée à une application topique sur la peau et en particulier sous forme d'émulsion huile-dans-eau, eau-dans-huile ou multiple (E/H/E ou H/E/H), qui peuvent éventuellement être des microémulsions ou des nanoémulsions, ou sous forme d'hydrodispersion, de solution, de gel aqueux ou de poudre. On préfère que cette composition se présente sous la forme d'une émulsion huile-dans-eau.

Cette composition est de préférence utilisée en tant que produit de soin ou de nettoyage de la peau du visage et/ou du corps et elle peut se présenter notamment sous forme de fluide, de gel ou de mousse, conditionnés par exemple dans un flacon-pompe, un aérosol ou un tube, ou de crème conditionnée par exemple dans un pot. En variante, elle peut avoir la forme d'un produit de maquillage et en particulier d'un fond de teint ou d'une poudre libre ou compacte.

De préférence, la composition mise en oeuvre selon l'invention est utilisée comme produit de soin du corps et elle se présente avantageusement sous forme de gel ou de fluide.

Elle peut contenir différents adjuvants, tels qu'au moins un composé choisi parmi :
- les huiles, qui peuvent être choisies notamment parmi : les huiles de silicone, linéaires ou cycliques, volatiles ou non volatiles, telles que les polydiméthylsiloxanes (dimethicones), les polyalkylcyclosiloxanes (cyclomethicones) et les polyalkylphenylsiloxanes (phenyldimethicones); les huiles synthétiques telles que les huiles fluorées, les alkyl benzoates et les hydrocarbures ramifiés tels que le polyisobutylène; les huiles végétales et notamment de soja ou de jojoba; et les huiles minérales telles que l'huile de paraffine;
- les cires, telles que l'ozokérite, la cire de polyéthylène, la cire d'abeille ou la cire de carnauba;
- les élastomères de silicone obtenus notamment par réaction, en présence d'un catalyseur, d'un polysiloxane ayant au moins un groupe réactif (hydrogène ou vinyle, notamment) et portant au moins un groupe alkyle (notamment méthyle) ou phényle, terminal et/ou latéral, avec un organosilicone tel qu'un organohydrogenpolysiloxane;
- les tensioactifs, de préférence émulsionnants, qu'ils soient non ioniques, anioniques, cationiques ou amphotères, et en particulier les esters d'acides gras et de polyols tel que les esters d'acides gras et de glycérol, les esters d'acides gras et de sorbitan, les esters d'acides gras et de polyéthylèneglycol et les esters d'acides gras et de sucrose; les éthers d'alcools gras et de polyéthylèneglycol; les alkylpolyglucosides; les polysiloxanes modifiés polyéthers; la bétaïne et ses dérivés; les polyquaterniums; les sels de sulfate d'alcools gras éthoxylés; les sulfosuccinates; les sarcosinates; les alkyl- et dialkylphosphates et leurs sels; et les savons d'acides gras;
- les co-tensioactifs tels que les alcools gras linéaires et en particulier les alcools cétylique et stéarylique;
- les épaississants et/ou gélifiants, et en particulier les homo- et copolymères réticulés ou non, hydrophiles ou amphiphiles, d'acide acryloylméthylpropane sulfonique (AMPS) et/ou d'acrylamide et/ou d'acide acrylique et/ou de sels ou d'esters d'acide acrylique; la gomme de xanthane ou de guar; les dérivés cellulosiques; et les gommes de silicone (dimethiconol);
- les humectants, tels que les polyols, dont la glycérine, le propylène glycol et les sucres, et les glycosaminoglycanes tels que l'acide hyaluronique et ses sels et esters;
- les filtres organiques, tels que les dérivés de dibenzoylméthane (dont le butyl methoxydibenzoylmethane), les dérivés d'acide cinnamique (dont l'ethylhexyl methoxycinnamate), les salicylates, les acides para-aminobenzoïques, les β-β'-diphénylacrylates, les benzophénones, les dérivés de benzylidène camphre, les phénylbenzimidazoles, les triazines, les phénylbenzotriazoles et les dérivés anthraniliques;
- les filtres inorganiques, à base d'oxydes minéraux sous forme de pigments ou de nanopigments, enrobés ou non, et en particulier à base de dioxyde de titane ou d'oxyde de zinc;
- les colorants;
- les conservateurs;
- les charges, et en particulier les poudres à effet soft-focus, qui peuvent être notamment choisies parmi les polyamides, la silice, le talc, le mica, les fibres (notamment de polyamide ou de cellulose);
- les tenseurs, et en particulier les protéines végétales, les latex synthétiques (notamment acryliques) et les dispersions colloïdales de charges inorganiques;
- les séquestrants tels que les sels d'EDTA;
- les parfums;
- et leurs mélanges, sans que cette liste ne soit limitative.

Des exemples de tels adjuvants sont cités notamment dans le Dictionnaire CTFA (International Cosmetic Ingredient Dictionary and Handbook publié par The Cosmetic, Toiletry and Fragrance Association, 9ème Edition, 2002).

La composition utilisée selon l'invention peut en outre comprendre d'autres actifs que ceux décrits précédemment et en particulier au moins un actif choisi parmi : les agents stimulant la production de facteurs de croissance ; les agents anti-glycation ou déglycants; les agents augmentant la synthèse de collagène ou prévenant sa dégradation (agents anti-collagénases, notamment inhibiteurs de métalloprotéinases matricielles); les agents augmentant la synthèse d'élastine ou prévenant sa dégradation (agents anti-élastases); les agents augmentant la synthèse de glycosaminoglycanes ou de protéoglycanes ou prévenant leur dégradation; les agents augmentant la prolifération ou la différenciation des kératinocytes; les agents augmentant la prolifération des fibloblastes; les agents dépigmentants, anti-pigmentants ou propigmentants; les agents anti-oxydants ou anti-radicalaires ou anti-pollution; les agents augmentant la synthèse de lipides épidermiques; les agents stimulant la lipolyse, inhibant la lipogénèse et/ou inhibant la différenciation des adipocytes; les agents drainants ou détoxifiants ou améliorant la microcirculation; et leurs mélanges, sans que cette liste ne soit limitative.

De préférence, la composition utilisée selon l'invention renferme au moins un agent stimulant la lipolyse e/ou au moins un agent stimulant la synthèse de collagène et plus préférentiellement une combinaison de ces agents.

Des exemples de tels actifs additionnels sont notamment : les extraits de plantes et en particulier les extraits de *Chondrus crispus,* de *Thermus thermophilus,* de *Pisum sativum,* de *Centella asiatica,* de *Sphacelaria scoparia, de Scenedesmus, de Moringa pterygosperma,* de lierre *(Hedera helix), de Castanea sativa, d'Hibiscus sabdriffa, de Polyanthes tuberosa, d'Argania spinosa,* de graines d*'Hibiscus esculentus, de Narcissus tarzetta,* de réglisse ou de *Ruscus esculatus;* une huile essentielle de *Citrus aurantium* (Neroli); les α-hydroxyacides tels que les acides glycolique, lactique et citrique, et leurs esters; les β-hydroxyacides, tels que l'acide salicylique et ses dérivés; les dérivés de silicium tels que le mannuronate de methylsilanol; les hydrolysats de protéines végétales (notamment de soja ou de noisette); les sucres ; les oligopeptides acylés, commercialisés notamment par la société SEDERMA sous les dénominations commerciales Maxilip®, Matrixyl® 3000, Biopeptide® CL ou Biopeptide® EL ou décrits dans la demande EP-1 449 517; les extraits de levure et en particulier de *Saccharomyces cerevisiae;* les extraits d'algues et en particulier de laminaires et de *Blidingia minima;* la caféine et les extraits de café et de maté en contenant; les vitamines et leurs dérivés tels que le palmitate de rétinyle, l'acide ascorbique, le glucoside d'ascorbyle, l'ascorbyl phosphate de magnésium ou de sodium, le palmitate d'ascorbyle, le tétraisopalmitate d'ascorbyle, le sorbate d'ascorbyle, le tocophérol, l'acétate de tocophéryle et le sorbate de tocophéryle; les homo- et copolymères de methacryloyloxyethylphosphorylcholine; l'urée; les céramides et les phopholipides; l'arbutine; l'acide kojique; l'acide ellagique; la dihydroxyacétone (DHA); et leurs mélanges.

L'invention sera maintenant illustrée par les exemples non limitatifs suivants.

### EXEMPLES

### Exemple 1 : Test de stimulation de la synthèse d'élastine

### a) Protocole

On a évalué l'effet d'un extrait de Bangle *(Zingiber cassumunar Roxb.)* sur la synthèse d'élastine par les fibroblastes, par Western Blot.

### Préparation de l'extrait

L'extrait testé a été obtenu selon le procédé suivant.

2.13 g de rhizomes de bengle *(Zingiber Purpureum Roscoe)* ont été broyés à l'aide d'un broyeur à couteaux (RETSCH) puis chargés dans un réacteur en verre de 20 litres équipé d'un reflux. On a ensuite ajouté 10 litres d'un mélange hexane/isopropanol (80/20 v/v) au broyat. On a laissé le mélange macérer à froid pendant deux heures, puis on l'a chauffé pendant cinq heures au reflux. Après filtration et vidange du réacteur, on a évaporé le solvant avec un évaporateur rotatif sous vide, pour récupérer 0.07694 kg d'oléorésine de bengle (rendement : 3.61%).

502 g de cette oléorésine ont été distillés par passage dans un appareil de distillation moléculaire du type KDL4 (UIC GmH) selon les paramètres de distillation suivants :

| **Paramètres de distillation** | |
|---|---|
| Débit d'insertion (ml/h) | 400 |
| Température évaporateur (°C) | 125 |
| Température condenseur (°C) | 65 |
| Température d'introduction du produit (°C) | 70 |
| Agitation (tours/min) | 200 |
| Pression vide (mbar) | 9,5.10⁻² |

Le culot de distillation a été récupéré puis soumis à un lavage avec de l'éthanol à 96.2° et du charbon actif, à une température de 50-60°C, en vue de le décolorer. Le filtrat ainsi obtenu a été soumis à une seconde opération de lavage dans les mêmes conditions. Le filtrat final a alors été filtré sur filtre conique pour éliminer les résidus de charbon actif, puis l'éthanol a été évaporé sous vide.

### Test in vitro

Des fibroblastes (Cambrex), ont été cultivés pour multiplication dans du milieu de culture adapté à la croissance de fibroblastes (DMEM supplémenté, GIBCO), puis les cultures de fibroblastes ont été ensemencées dans des plaques à 6 puits avant traitement par des concentrations différentes d'extrait de Bengle.

Les surnageants correspondant à un nombre de cellules équivalent et à une teneur équivalente en protéines cellulaires, ont été fractionnés par gel PAGE avec un gradient de 8 à 18% (Amersham Pharmacia Biotech, Uppsala, Suède) et transférés sur des membranes en nitrocellulose (Schleicher und Schüll, Dassel, Allemagne). On a ensuite procédé à une immunodétection à l'aide d'un anticorps anti-élastine monoclonal (Elastin Products Co, PR 533), suivie d'un antisérum d'IgG de lapin anti-chèvre conjugué à l'HRP (Amersham). Les produits de la réaction ont été détectés par chimioluminescence à l'aide du kit ECL (Amersham), selon les instructions du fabricant, et quantifiés par analyse d'image.

Résultats : testé à 50 µg/ml (0.005%) et 100 µg/ml (0.01%), l'extrait de Bengle augmente la synthèse d'élastine de 238% en moyenne (par rapport au témoin non traité) ± 70% et de 161% en moyenne ± 31%, respectivement.

Il est ainsi possible d'appliquer topiquement sur la peau cet extrait pour lutter contre les signes cutanés du vieillissement.

### Exemple 2 : Ccmpositions cosmétiques

Les compositions suivantes peuvent être préparées de façon classique pour l'homme du métier. Les quantités indiquées ci-dessous sont exprimées en pourcentages pondéraux.

**Emulsion H/E**

| | |
|---|---|
| Extrait de Bengle* | 0.01 % |
| Methylsilanol mannuronate | 5.00 % |
| Emulsionnant (steareth-21) | 1.50 % |
| Tocopherol acetate | 0.50 % |
| Huiles végétales | 5.00 % |
| Huiles siliconées | 6.00 % |
| Octyldodecanol | 2.00 % |
| Glycérine | 5.00 % |
| Gélifiants | 2.80 % |
| Alcool dénaturé | 5.00 % |
| Séquestrant | 0.05 % |
| Ajusteur de pH | qs |
| Conservateurs | qs |
| Eau | qsp 100.00 % |

| | |
|---|---|
| * préparé comme décrit à l'Exemple 1 | |

Cette émulsion peut être appliquée tous les soirs sur le corps pour le raffermir.

**Gel aqueux**

| | |
|---|---|
| Caféine | 5.00 % |
| Extrait de Bengle* | 0.10 % |
| Polyisobutène hydrogéné | 5.00 % |
| Emulsionnants | 5.00 % |
| Alcool dénaturé | 15.00 % |
| Acides gras | 1.50 % |
| Séquestrant | 0.05 % |
| Butylène glycol | 3.00 % |
| Gélifiants | 5.00 % |
| Ajusteur de pH | qs |
| Conservateurs | qs |
| Eau | qsp 100.00 % |

| | |
|---|---|
| * préparé comme décrit à l'Exemple 1 | |

Ce gel peut être appliqué matin et soir sur le corps pour le raffermir et atténuer les capitons.

## Revendications

1. Procédé cosmétique de soin de la peau, destiné à prévenir et/ou traiter au moins un signe cutané du vieillissement, comprenant l'application topique sur la peau d'une composition renfermant au moins un actif choisi parmi une cassumunarine, un arylbuténoïde et un extrait botanique en contenant.

2. Procédé selon la revendication 1, **caractérisé en ce que** les cassumunarines sont choisies parmi les cassumunarines A, B et C.

3. Procédé selon la revendication 1 ou 2, **caractérisé en ce que** les arylbuténoïdes répondent à la formule (I) ci-dessous : 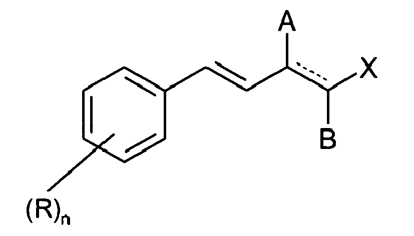 dans laquelle :
n désigne un nombre entier allant de 1 à 3 ;
R désigne un groupe hydroxy ou méthoxy, les groupes R pouvant être identiques ou différents dans le cas où n vaut 2 ou 3 ;
A désigne un atome d'hydrogène ou un groupe hydroxy et X désigne un atome d'hydrogène ou un groupe hydroxy ou acétoxy, ou A et X désignent chacun un atome de carbone et forment ensemble un cycle à six chaînons éventuellement insaturé, comportant éventuellement un ou plusieurs substituants (R'O)ₘAr où Ar est un groupe phényle, R' est un atome d'hydrogène ou un groupe acétyl et m est un nombre entier allant de 1 à 3 ; et
les pointillés désignent une liaison qui peut ou non être présente.

4. Procédé selon la revendication 3, **caractérisé en ce que** n est égal à 2.

5. Procédé selon la revendication 4, **caractérisé en ce que** les groupes R sont disposés en positions méta et para sur le cycle.

6. Procédé selon l'une quelconque des revendications 3 à 5, **caractérisé en ce que** A et X désignent chacun un atome d'hydrogène.

7. Procédé selon l'une quelconque des revendications 3 à 5, **caractérisé en ce que** A et X forment ensemble un cycle substitué par un groupe diméthoxyphényle.

8. Procédé selon la revendication 3, **caractérisé en ce que** les arylbuténoïdes sont choisis parmi : la 4-(4-hydroxy-3-methoxyphenyl)-3-buten-2-one, le (E)-1-(,3,4-diméthoxyphényl)but-1-ène, le (E)-4-(3,4-diméthoxyphényl)buta-1,3-diène, le (E)-4-(3,4-diméthoxyphényl)but-3-én-1-yl acétate, le (E)-4-(3,4-diméthoxyphényl)but-3-èn-1-ol, le (E)-3-hydroxy-1-(3,4-diméthoxyphényl)but-1-ène, le (E)-4-(4-hydroxy-3-méthoxyphényl)but-3-èn-1-yl acétate et le 4-(2,4,5-trimethoxyphenyl)but-1,3-diène.

9. Procédé selon l'une quelconque des revendications 1 à 3, **caractérisé en ce que** l'extrait botanique est un extrait de *Zingiber cassumunar Roxb.*

10. Procédé selon l'une quelconque des revendications 1 et 9, **caractérisé en ce que** l'extrait est préparé par extraction à l'aide d'au moins un solvant apolaire ayant un indice de polarité inférieur à 1, éventuellement en mélange avec au moins un solvant polaire ayant un indice de polarité supérieur à 3.5.

11. Procédé selon la revendication 10, **caractérisé en ce que** le solvant apolaire est choisi parmi : l'hexane, le cyclohexane, l'heptane et l'isooctane,

12. Procédé selon la revendication 10 ou 11, **caractérisé en ce que** le solvant organique polaire est un solvant alcoolique, tel que l'éthanol ou l'isopropanol.

13. Procédé selon l'une quelconque des revendications 10 à 12, **caractérisé en ce que** le ratio du solvant apolaire au solvant polaire est compris entre 50:50 et 95:5 et va de préférence de 70:30 à 90:10, plus préférentiellement de 75:25 à 85:15.

14. Procédé selon l'une quelconque des revendications 1 à 13, **caractérisé en ce qu'**il est destiné à lutter contre les signes cutanés liés au ralentissement de la synthèse, ou à la dégradation, de l'élastine et en particulier à prévenir et/ou traiter la perte de fermeté et/ou d'élasticité de la peau et/ou les rides.

15. Procédé selon l'une quelconque des revendications 1 à 14, **caractérisé en ce que** la composition est appliquée sur le corps humain, à l'exclusion du visage, et notamment sur les genoux, les cuisses, les hanches, les fesses, le ventre et/ou les bras.

16. Utilisation cosmétique d'une cassumunarine et/ou d'un arylbuténoïde et/ou d'un extrait botanique en contenant, pour prévenir et/ou traiter au moins un signe cutané du vieillissement.
